# EUROPEAN PATENT SPECIFICATION

(11) **EP 2 979 721 B1**
(45) Date of publication and mention of the grant of the patent: **28.12.2016**
(21) Application number: 15170289.1
(22) Date of filing: 02.06.2015
(51) Int. Cl.: A61M 25/10, A61M 25/01, A61M 25/00

(54) **CATHETER**
KATHETER
CATHÉTER

(30) Priority: 29.07.2014 JP 2014153952
(43) Date of publication of application: 03.02.2016
(60) Divisional application: 16193271.0
(73) Proprietor: ASAHI INTECC CO., LTD., Nagoya-shi, Aichi 463-0024 (JP)
(72) Inventor: Nishigishi, Makoto c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 1 676 594
- WO-A1-2010/048676
- WO-A1-2012/068505
- US-A1- 2013 211 385

## Description

### TECHNICAL FIELD

The present invention relates to a catheter capable of efficiently transmitting operator's pushing force to the distal end and capable of bending along curved regions of a blood vessel, bile duct, pancreatic duct and the like.

### BACKGROUND ART

When a narrowed or obstructed segment is formed in a blood vessel, bile duct, pancreatic duct and the like, the flow of blood, bile (gall), pancreatic fluid and the like may be hindered. Traditionally, treatments with a catheter have been widely performed to treat a narrowed or obstructed segment. In order to allow a catheter to reach a narrowed or obstructed segment, operator's pushing force needs to be efficiently transmitted to the distal end of a catheter, and a catheter should be less often caught up at a curved region of a blood vessel, bile duct, pancreatic duct and the like along the way.

In this context, as a method of improving pushing force toward the distal end of a catheter, known is a catheter in which a core wire is axially disposed between an outer tube and an inner tube (For example, see WO2006/126642 and JP 2012-223207). One core wire is disposed in the catheter according to Patent Literature 1, and multiple core wires are disposed in the catheter according to Patent Literature 2.

However, in the catheters according to WO2006/126642 and JP 2012-223207, a core wire is fixed to other members (for example, an outer tube, an inner tube, a hypo tube or the like), showing almost no degree of freedom. Therefore, there have been the following problems: when a catheter is inserted into a blood vessel, bile duct, pancreatic duct and the like, a core wire can not move to the optimal position along a curved region of the blood vessel, bile duct, pancreatic duct and the like, resulting in a breakage of the core wire along the way; and a catheter may be caught up at a curved region of a blood vessel, bile duct, pancreatic duct and the like along the way, preventing the catheter from reaching a narrowed or obstructed segment. In particular, although axial rigidity can be improved when multiple core wires are disposed, the multiple core wires may interfere each other when the catheter is inserted up to a curved region of a blood vessel, bile duct, pancreatic duct and the like. This may cause a significant problem that a catheter can not bend along the blood vessel, bile duct, pancreatic duct and the like, preventing the catheter from reaching a narrowed or obstructed segment.

WO 2010/048676 describes a catheter assembly comprising a push wire and a pull wire. The distal end of the pull wire is anchored to the distal end of the steering tube. Further, the push wire is received in the tip element and the proximal end of the push wire is attached to the displacement device. Further, WO 2010/048676 discloses the second tubular member including a proximal end portion defining a proximal end.

WO2012/068505 describes an electrode catheter comprising an inner layer and an outer layer comprising a braided wire assembly for direct current tissue therapies. The outer layer further includes a plurality of minor lumens coupled thereto.

US2013/0211385 refers to a body cavity drainage devices comprising a drainage tube, an activation device attached to the proximal end of the drainage tube, and first and second flexible members disposed within closed lumens within a wall of the drainage tube. The activation device moves the first and second flexible members to change the shape of the drainage tube.

EP1676594 discloses a catheter comprising a shaft, and a balloon attached to the distal portion of the shaft.

### SUMMARY OF THE INVENTION

### TECHNICAL PROBLEM

The present invention is made in view of these circumstances. An object of the present invention is to provide a catheter as described in the claims in which an operator's pushing force can be efficiently transmitted to the distal end and in which the risk of breakage of a core wire and of being caught up at a curved region of a blood vessel, bile duct, pancreatic duct and the like along the way can be reduced.

### SOLUTION TO PROBLEM

The above object can be solved by the means listed below.

An aspect 1 of the present invention is a catheter comprising an outer tube, a first inner tube inserted into the outer tube, a second inner tube inserted into the outer tube parallel to the first inner tube, the second inner tube inserted from the middle through the distal end of the catheter and having an insertion opening provided at a proximal end and a distal end opening provided at a distal end of the second inner tube, a first core wire inserted between the outer tube and the first inner tube, the first core wire being fixed to the outer tube or the first inner tube and a second core wire inserted parallel to the first core wire, the second core wire being capable of moving between the outer tube and the first inner tube. Further, the first core wire is longer than the second core wire, and a distal end of the first core wire is located in a distal end side relative to the insertion opening of the second inner tube while a distal end of the second core wire is located in a proximal end side relative to the insertion opening of the second inner tube.

An aspect 2 of the present invention is the catheter according to the aspect 1, having a portion where a cross sectional area of the second core wire is smaller than that of the first core wire.

An aspect 3 of the present invention is the catheter according to the aspects 1 or 2, wherein a restriction part restricting the movement of the second core wire toward the distal end is provided at the first core wire or the second core wire.

An aspect 4 of the present invention is the catheter according to the aspect 3, wherein the restriction part is provided at the second core wire, and the restriction part is positioned in the proximal side relative to the proximal end of the first core wire or the proximal end of the outer tube.

### ADVANTAGEOUS EFFECT OF THE INVENTION

In the catheter according to the aspect 1 of the present invention, disposed are the first core wire fixed to the outer tube or the inner tube between the outer tube and the inner tube, and the second core wire parallel to the first core wire and capable of moving between the outer tube and the inner tube. By providing the first core wire fixed to the outer tube or the inner tube and the movable second core wire, axial rigidity can be improved to efficiently transmit operator's pushing force to the distal end. Further, the second core wire can move to the optimal position when the catheter is inserted up to a curved region of a blood vessel, bile duct, pancreatic duct and the like. Therefore, a risk can be reduced that the first core wire and the second core wire interfere each other to prevent the catheter from not bending along the blood vessel, bile duct, pancreatic duct and the like, resulting in breakage of any one or both of the first core wire and the second core wire.

The catheter according to the aspect 2 of the present invention has a portion where a cross sectional area of the second core wire is smaller than that of the first core wire. Therefore, the second core wire can easily move to the optimal position even in a case where the shape of the catheter is deformed due to external force exerted on the catheter when it is inserted up to a curved region of a blood vessel, bile duct, pancreatic duct and the like. As a result, a risk can be further reduced that the catheter is caught up in a curved region of the blood vessel, bile duct, pancreatic duct and the like along the way.

In the catheter according to the aspect 3 of the present invention, the restriction part restricting the movement of the second core wire toward the distal end is provided at the first core wire or the second core wire. Therefore, a risk that the second core wire is projected out of the outer tube when an operator pushes the catheter in the direction toward the distal end can be reduced by providing the restriction part. Moreover, a force which drives the second core wire in the direction toward the distal end can be transmitted to the outer tube or the inner tube through the restriction part, which can in turn improve a force to push the catheter.

In the catheter according to the aspect 4 of the present invention, the restriction part is provided at the second core wire, and the restriction part is positioned at the proximal side relative to the proximal end of the first core wire or the proximal end of the outer tube. Therefore, a risk can be further reduced that the second core wire is projected out of the outer tube since the restriction part provided at the second core wire can be caught up at the proximal end of the first core wire or the proximal end of the outer tube when an operator pushes the catheter in the direction toward the distal end.

### BRIEF DESCRIPTION OF THE DRAWINGS

Fig. 1 shows an overall view of a catheter according to an embodiment of the present invention.
Figs. 2A to 2C show the A-A cross section, the B-B cross section and the C-C cross section in Fig. 1, respectively.
Figs. 3A to 3D show how the second core wire moves in a circumferential direction when external force is exerted to a catheter.
Fig. 4 shows an overall view of a catheter according to a second embodiment of the present invention.
Fig. 5 shows the D-D cross section in Fig. 4.
Fig. 6 shows an overall view of a catheter according to a third embodiment of the present invention.
Fig. 7 shows the E-E cross section in Fig. 6.
Fig. 8 shows an overall view of a catheter according to a fourth embodiment of the present invention.
Fig. 9 shows the F-F cross section in Fig. 8.
Fig. 10 shows an overall view of a balloon catheter according to a fifth embodiment of the present invention.

### DESCRIPTION OF EMBODIMENTS

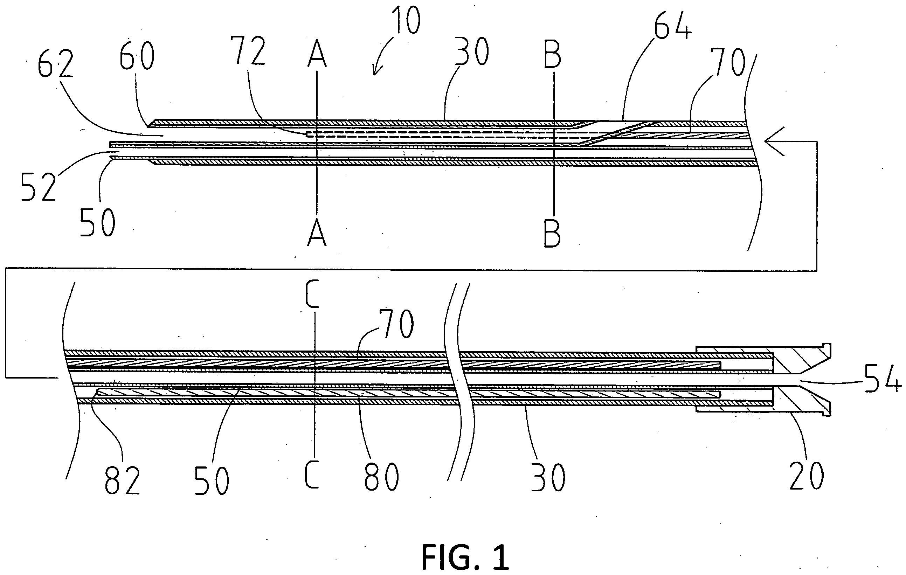

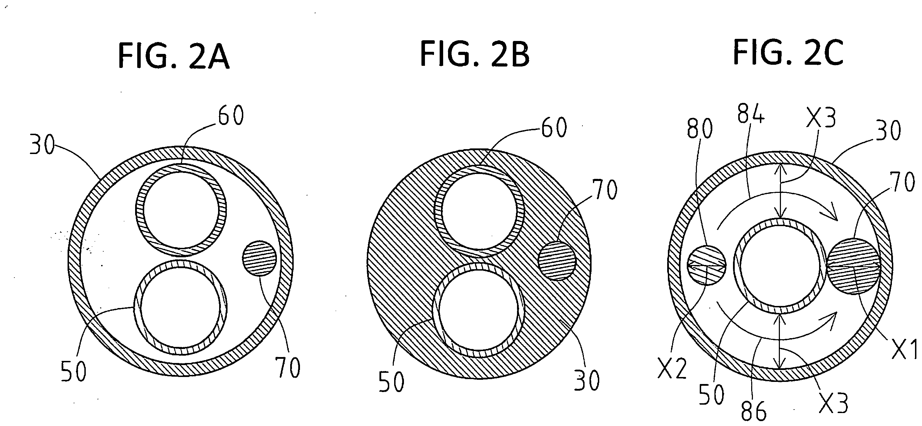

First, an example is described with reference to Figs. 1 to 3 in which a catheter 10 according to an embodiment of the present invention is used. The left side in Figs. 1 and 2 corresponds to the distal end side (the front side) which is to be inserted into the body, and the right side corresponds to the proximal side (the base end side) which is to be operated by an operator such as a physician.

The catheter 10 may be used for treating, for example, a narrowed or obstructed segment formed in a blood vessel, bile duct, pancreatic duct and the like. As shown in Fig. 1, the catheter 10 mainly comprises a connector 20, an outer tube 30, a first inner tube 50, a second inner tube 60, a first core wire 70 and a second core wire 80.

The first inner tube 50 is inserted into the outer tube 30 throughout the almost full length of the catheter 10. A guide wire may be inserted into the first inner tube 50. In order to allow for easy insertion of the guide wire, the connector 20 is connected to the proximal end of the outer tube 30 and the proximal end of the first inner tube 50. A first distal end opening 52 is provided at the distal end of the first inner tube 50, and a first insertion opening 54 is provided at the proximal end of the first inner tube 50 through the connector 20.

The second inner tube 60 is inserted into the outer tube 30 parallel to the first inner tube 50 from the middle through the distal end of the catheter 10. As in the first inner tube 50, a guide wire may also be inserted into the second inner tube 60. A second distal end opening 62 is provided at the distal end of the second inner tube 60, and a second insertion opening 64 is provided at the proximal end of the second inner tube 60.

The outer tube 30, the first inner tube 50 and the second inner tube 60 are formed with a thermoplastic resin. For example, a resin such as polyamide, polyamide elastomer, polyolefine, polyester, polyester elastomer and nylon can be used.

The first inner tube 50 extends throughout the almost full length of the catheter 10. Therefore, disadvantageously, an operator can not easily replace a guide wire which was inserted into the first inner tube 50. On the other hand, advantageously, the rigidity of the catheter 10 is improved by a guide wire when the guide wire is inserted into the first inner tube 50, allowing an operator to easily push the catheter 10 toward the distal end. Moreover, the second inner tube 60 merely extends from the middle to the distal end of the catheter 10. Therefore, advantageously, an operator can easily replace a guide wire which was inserted into the second inner tube 60. On the other hand, disadvantageously, the rigidity is improved only at the distal end side of the catheter 10 even when a guide wire is inserted into the second inner tube 60, and the catheter 10 may break near the second insertion opening 64 of the second inner tube 60 where the rigidity suddenly changes when an operator pushes the catheter 10 in the direction toward the distal end. The catheter 10 comprises both the first inner tube 50 and the second inner tube 60. Therefore, an operator can readily replace a second guide wire inserted into the second inner tube 60 while maintaining a state where a first guide wire is inserted into the first inner tube 50, and at the same time can easily push the catheter 10 in the direction toward the distal end.

As shown in Fig. 1, the first core wire 70 and the second core wire 80 made of metal and extending in the axial direction are inserted into the catheter 10 between the outer tube 30 and the first inner tube 50. The first core wire 70 and the second core wire 80 each have a circular cross section and are a tapered metal wire member in which the diameter becomes smaller toward the distal end. There is no particular limitation for the material of the first core wire 70 and the second core wire 80, and for example, stainless steel and a superelastic alloy such as an Ni-Ti alloy can be used.

The first core wire 70 is a metal wire member longer than the second core wire 80. Therefore, a distal end 72 of the first core wire 70 is located in the distal end side relative to the second insertion opening 64 of the second inner tube 60 while a distal end 82 of the second core wire 80 is located in the proximal end side relative to the second insertion opening 64 of the second inner tube 60.

Figs. 2A to 2C show the A-A cross section, the B-B cross section and the C-C cross section in Fig. 1, respectively. The first core wire 70 is fixed with the outer tube 30 near the second insertion opening 64 of the second inner tube 60 (see Fig. 2B). Therefore, the first core wire 70 does not move in the axial and circumferential directions when an operator operates the catheter 10.

The second core wire 80 inserted parallel to the first core wire 70 is not fixed to the outer tube 30 and the first inner tube 50 (see Fig. 1 and Fig. 2C). Therefore, the second core wire 80 can move in the axial and circumferential directions between the outer tube 30 and the first inner tube 50 when an operator operates the catheter 10. As shown in Fig. 2C, the second core wire 80 can move in either of the clockwise direction as shown by an arrow 84 or the counterclockwise direction as shown by an arrow 86.

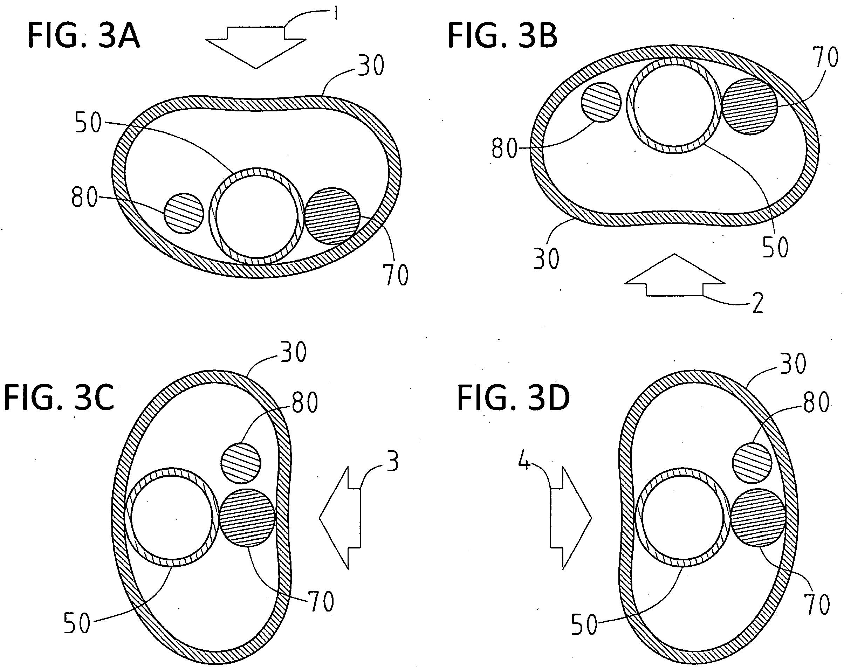

Figs. 3A to 3D show how the second core wire 80 moves in a circumferential direction when external forces 1, 2, 3 and 4 are exerted on the catheter by a curved region of a blood vessel, bile duct, pancreatic duct and the like. Figs. 3A to 3D show the C-C cross section in Fig. 1 as in Fig. 2C. As shown in Figs. 3A and 3B, when the external forces 1 and 2 are exerted on the catheter 10 by a curved region of a blood vessel, bile duct, pancreatic duct and the like, the outer tube 30 changes into a horizontally elongated ellipsoidal shape due to the external forces 1 and 2. Therefore, the second core wire 80 tends to move to, for example, a position where the first core wire 70 and the first inner tube 50 are horizontally aligned (in other words, a symmetrical position to the first core wire 70 relative to the first inner tube 50). Meanwhile, as shown in Figs. 3C and 3D, when the external forces 3 and 4 are exerted on the catheter 10 by a curved region of a blood vessel, bile duct, pancreatic duct and the like, the outer tube 30 changes into a vertically elongated ellipsoidal shape due to the external forces 3 and 4. Therefore, the second core wire 80 tends to move to, for example, a position above the first core wire 70 and the first inner tube 50.

As described above, the catheter 10 has a configuration in which the second core wire 80 can move to the optimal position depending on the external forces 1, 2, 3 and 4 exerted at a curved region of the blood vessel, bile duct, pancreatic duct and the like when the catheter 10 is inserted to the curved region of a blood vessel, bile duct, pancreatic duct and the like. Therefore, a risk can be reduced that the first core wire 70 and the second core wire 80 interfere each other to prevent the catheter 10 from not bending along a curved region of a blood vessel, bile duct, pancreatic duct and the like, resulting in breakage of any one or both of the first core wire 70 and the second core wire 80. Moreover, the first core wire 70 fixed to the outer tube 30 and the second core wire 80 not fixed to the outer tube 30 are disposed between the outer tube 30 and the first inner tube 50 in the catheter 10. Therefore, the axial rigidity can be improved, allowing the pushing force from an operator to be efficiently transmitted to the distal end. As a result, the catheter 10 can be easily delivered to a narrowed or obstructed segment.

Further, as shown in Fig. 2C, when X1 represents the diameter of the first core wire 70, and X2 represents the diameter of the second core wire 80, and X3 represents a distance between the outer tube 30 and the inner tube 50, there is a portion where the diameter X1 of the first core wire 70 is equal to the distance X3 between the outer tube 30 and the first inner tube 50 (X1 = X3) while the diameter X2 of the second core wire 80 is smaller than the distance X3 between the outer tube 30 and the first inner tube 50 (X2 < X3). Therefore, the frictional resistance between the second core wire 80 and the outer tube 30 and the frictional resistance between the second core wire 80 and the inner tube 50 can be reduced when the second core wire 80 moves depending on the external forces 1, 2, 3 and 4 exerted at a curved region of a blood vessel, bile duct, pancreatic duct and the like. Since this allows the second core wire 80 to smoothly move to the optimal position, a risk can be further reduced that the catheter 10 is caught up at a curved region of a blood vessel, bile duct, pancreatic duct and the like along the way.

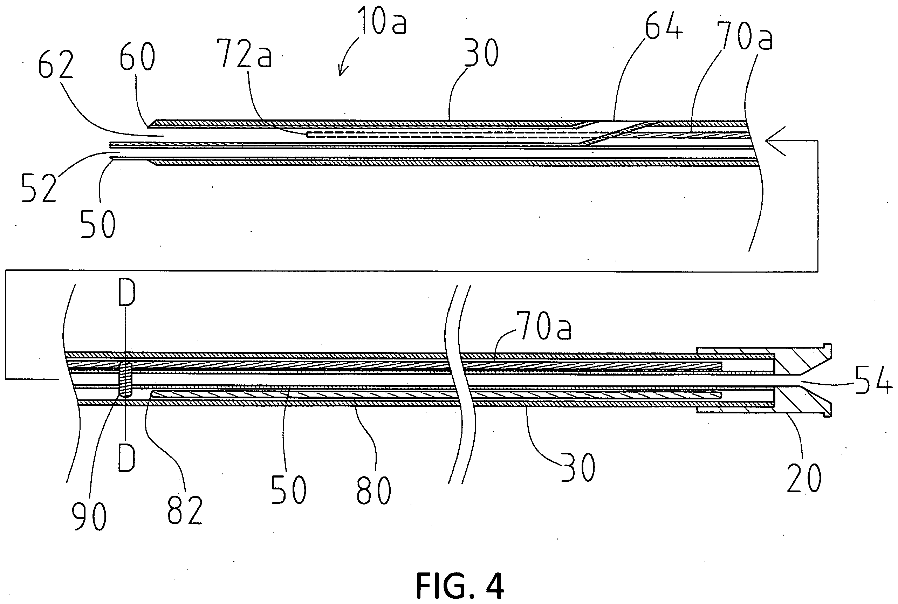

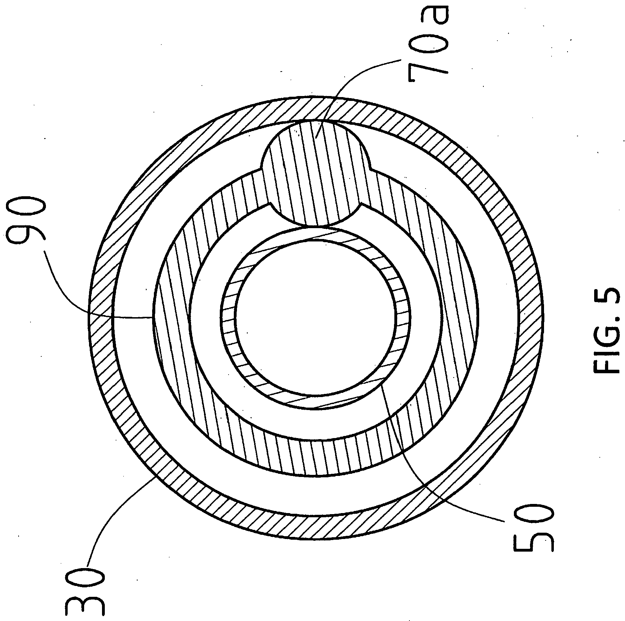

Next, a catheter 10a according to a second embodiment of the present invention is described with reference to Figs. 4 and 5. Note that as in Fig. 1, the left side in Figs. 4 corresponds to the distal end side (the front side) which is to be inserted into the body, and the right side corresponds to the proximal side (the base end side) which is to be operated by an operator such as a physician. Note that Fig. 5 shows the D-D cross section in Fig. 4.

Only differences from the catheter 10 shown in Figs. 1 to 3 are described. As shown in Figs. 4 and 5, the catheter 10a comprises an annular restriction part 90 in the middle of a first core wire 70a. The restriction part 90 is located in the distal end side relative to the distal end 82 of a second core wire 80, and restricts the movement of the distal end of the second core wire 80 in the direction toward the distal end. Note that the distal end 72a of a first core wire 70a is located in the distal end side relative to a second insertion opening 64 of a second inner tube 60 as in the catheter 10.

There is no particular limitation for the material of the restriction part 90, but the restriction part 90 of the catheter 10a is formed with the same metal material as the first core wire 70a. Specifically, the restriction part 90 is formed by welding an annular member comprising the same metal material as the first core wire 70a to the first core wire 70a. There is no particular limitation for the method of forming the restriction part 90, and the restriction part 90 may be formed, for example, by bonding an annular member comprising a resin material to the first core wire 70a using an adhesive agent.

Moreover, the restriction part 90 may be formed by melt-welding the outer tube 30 with the first inner tube 50 without using other members as long as the restriction part 90 can restrict the movement of the second core wire 80 in the direction toward the distal end by making contact with the distal end 82 of the second core wire 80.

As described above, the movement of the second core wire 80 in the direction toward the distal end can be restricted in the case of the catheter 10a since the restriction part 90 makes contact with the distal end 82 of the second core wire 80 when an operator pushes the catheter 10a in the direction toward the distal end. Therefore, a risk that the distal end 82 of the second core wire 80 is projected out of the outer tube 30 and a risk that it breaks through the second inner tube 60 can be reduced. Moreover, a force which drives the second core wire 80 to move in the direction toward the distal end is transmitted to the first core wire 70a through the restriction part 90. Since the first core wire 70a in the catheter 10a is fixed to the outer tube 30 as in the catheter 10 (see Fig. 2B), a force transmitted through the restriction part 90 can be transmitted to the outer tube 30 through the first core wire 70a. As a result, the force to push the catheter 10a can be further improved.

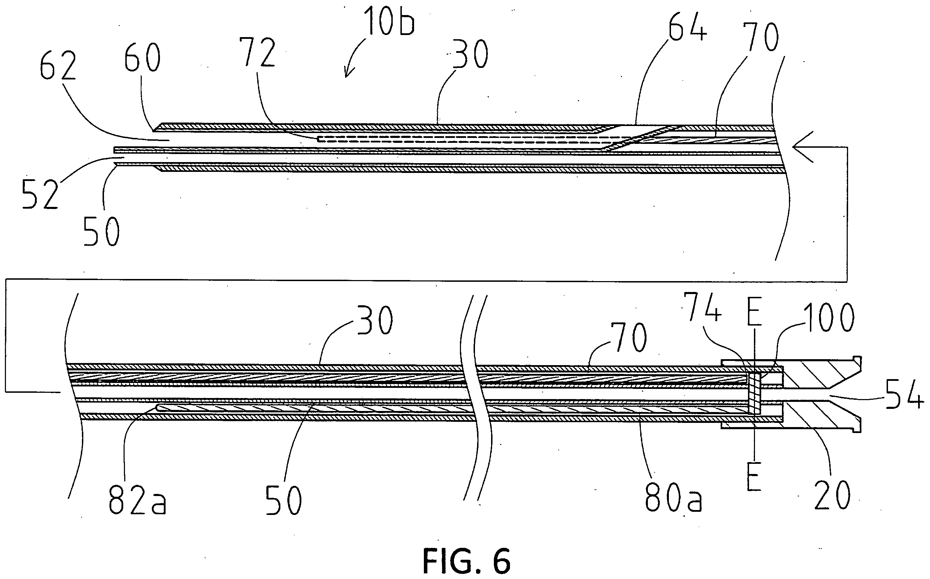

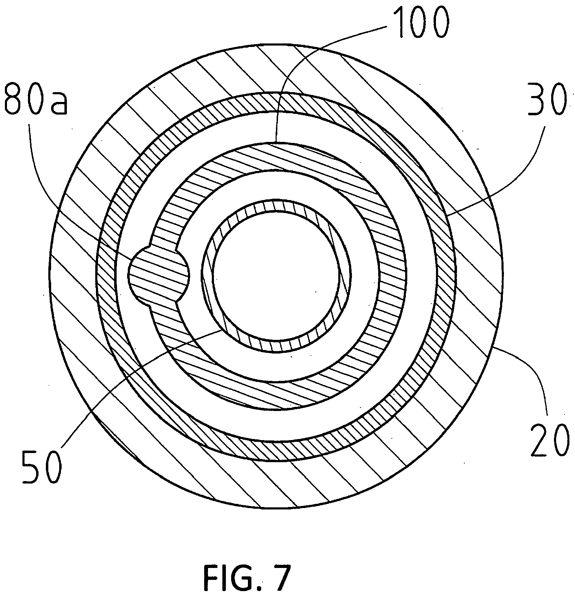

Next, the catheter 10b according to a third embodiment of the present invention is described with reference to Figs. 6 and 7. Note that as in Fig. 1, the left side in Fig. 6 corresponds to the distal end side (the front side) which is to be inserted into the body, and the right side corresponds to the proximal side (the base end side) which is to be operated by an operator such as a physician. Note that Fig. 7 shows the E-E cross section in Fig. 6.

Only differences from the catheter 10 shown in Figs. 1 to 3 are described. As shown in Figs. 6 and 7, the catheter 10b comprises an annular restriction part 100 at the proximal end of a second core wire 80a. The restriction part 100 makes contact with the proximal end 74 of the first core wire 70, and restricts the movement of the second core wire 80a in the direction toward the distal end.

There is no particular limitation for the material of the restriction part 100, but the restriction part 100 of the catheter 10b is formed with the same metal material as the second core wire 80a. Specifically, the restriction part 100 is formed by welding an annular member comprising the same metal material as the second core wire 80a to the proximal end of the second core wire 80a. There is no particular limitation for the method of forming the restriction part 100, and the restriction part 100 may be formed, for example, by bonding an annular member comprising a resin material to the second core wire 80a using an adhesive agent.

Moreover, the restriction part 100 may be formed by winding the proximal end of the second core wire 80a around the outer periphery of the first inner tube 50 without using other members in the proximal side relative to the proximal end 74 of the first core wire 70 as long as the restriction part 100 can restrict the movement of the second core wire 80a in the direction toward the distal end by making contact with the proximal end 74 of the first core wire 70.

As described above, in the case of the catheter 10b, the restriction part 100 provided at the second core wire 80a makes contact with the proximal end 74 of the first core wire 70 when an operator pushes the catheter 10b in the direction toward the distal end. Therefore, the second core wire 80a may be restricted not to move in the direction toward the distal end. Therefore, a risk that the distal end 82a of the second core wire 80a is projected out of the outer tube 30 and a risk that it breaks through the second inner tube 60 can be reduced. Moreover, a force which drives the second core wire 80a to move in the direction toward the distal end is transmitted to the first core wire 70 through the restriction part 100. Since the first core wire 70 in the catheter 10b is fixed to the outer tube 30 as in the catheter 10 (see Fig. 2B), a force transmitted through the restriction part 100 can be transmitted to the outer tube 30 through the first core wire 70. As a result, the force to push the catheter 10b can be further improved. Further, since the restriction part 100 of the catheter 10b is provided within the connector 20, the restriction part 100 is not inserted into the blood vessel, bite duct, pancreatic duct and the like. Therefore, a risk can be further reduced that the catheter 10b is caught up in a curved region of the blood vessel, bile duct, pancreatic duct and the like along the way because of the restriction part 100.

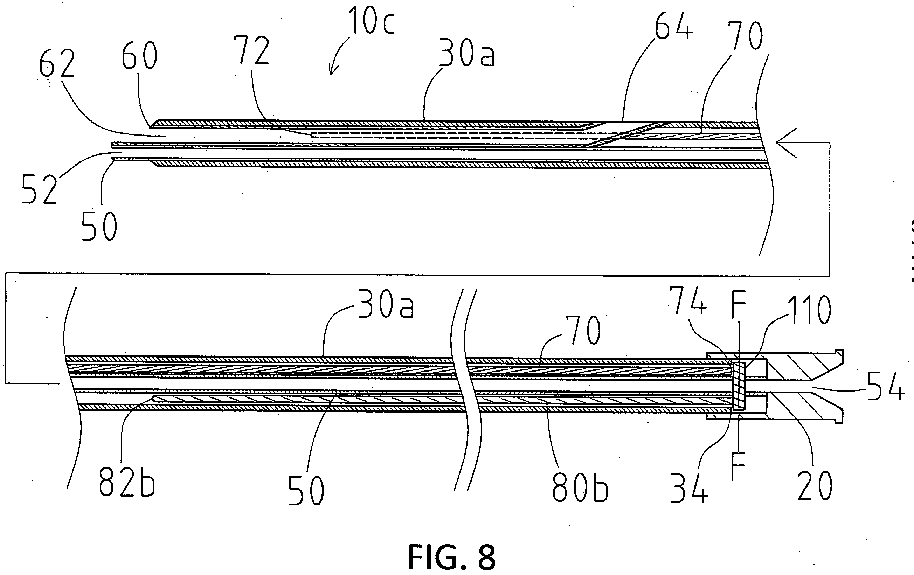

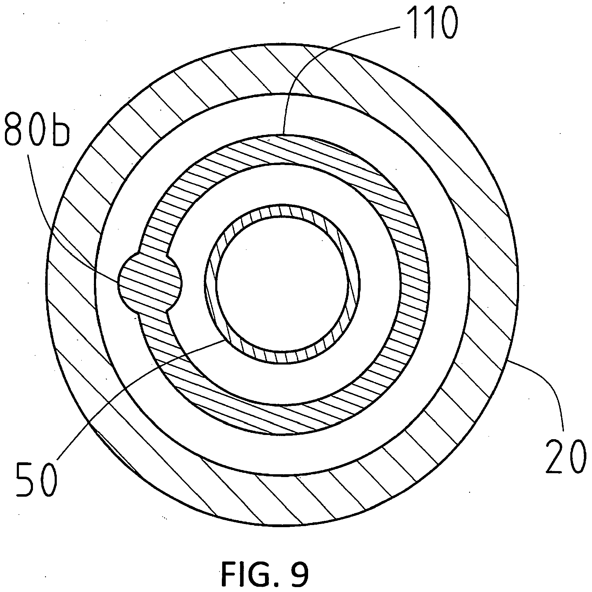

Next, a catheter 10c according to a fourth embodiment of the present invention is described with reference to Figs. 8 and 9. Note that as in Fig. 6, the left side in Fig. 8 corresponds to the distal end side (the front side) which is to be inserted into the body, and the right side corresponds to the proximal side (the base end side) which is to be operated by an operator such as a physician. Note that Fig. 9 shows the F-F cross section in Fig. 8.

Only differences from the catheter 10b shown in Figs. 6 and 7 are described. As shown in Figs. 8 and 9, the catheter 10c comprises an annular restriction part 110 at the proximal end of a second core wire 80b. The restriction part 110 makes contact the proximal end 74 of a first core wire 70 and the proximal end 34 of an outer tube 30a to restrict the movement of the second core wire 80b in the direction toward the distal end.

There is no particular limitation for the material of the restriction part 110. The restriction part 110 in the catheter 10c is formed with the same metal material as the second core wire 80b as in the catheter 10b. Specifically, the restriction part 110 is formed by welding an annular member comprising the same metal material as the second core wire 80b to the proximal end of the second core wire 80b. There is no particular limitation for the method of forming the restriction part 110, and the restriction part 110 may be formed, for example, by bonding an annular member comprising a resin material to the second core wire 80b using an adhesive agent.

Moreover, the restriction part 110 may be formed by winding the proximal end of the second core wire 80b around the outer periphery of the first inner tube 50 without using other members in the proximal side relative to the proximal end 74 of the first core wire 70 and the proximal end 34 of the outer tube 30a as long as the restriction part 110 can restrict the movement of the second core wire 80b in the direction toward the distal end by making contact with the proximal end 74 of the first core wire 70 and the proximal end 34 of the outer tube 30a.

As described above, in the case of the catheter 10c, the second core wire 80b may be restricted not to move in the direction toward the distal end since the restriction part 110 provided at the second core wire 80b makes contact with the proximal end 74 of the first core wire 70 when an operator pushes the catheter 10c in the direction toward the distal end. Therefore, a risk that the distal end 82b of the second core wire 80b is projected out of the outer tube 30a and a risk that it breaks through the second inner tube 60 can be reduced. Moreover, a force which drives the second core wire 80b to move in the direction toward the distal end can be transmitted to the first core wire 70 as well as the outer tube 30a through the restriction part 110. As a result, the force to push the catheter 10c can be further improved.

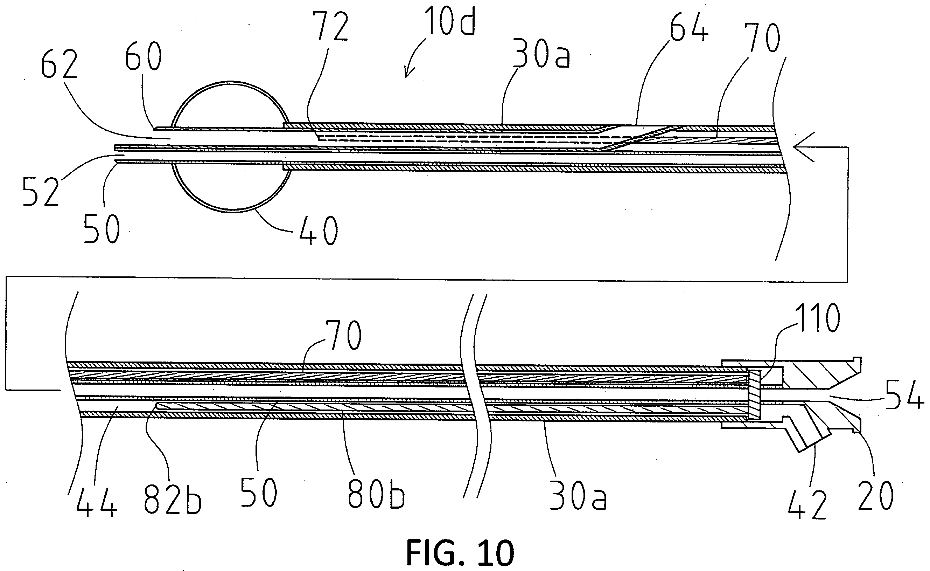

Next, a balloon catheter 10d according to a fifth embodiment of the present invention is described with reference to Fig. 10. Note that as in Fig. 8, the left side in Fig. 10 corresponds to the distal end side (the front side) which is to be inserted into the body, and the right side corresponds to the proximal side (the base end side) which is to be operated by an operator such as a physician.

Only differences from the catheter 10c shown in Figs. 8 and 9 are described. As shown in Fig. 10, the balloon catheter 10d comprises a balloon 40 melt-welded to the distal end of an outer tube 30a. The distal end of the balloon 40 is melt-welded to a first inner tube 50 and a second inner tube 60.

A supply opening 42 for supplying a liquid such as a contrast medium and physiological saline is provided at a connector 20, and an indeflator (not shown) may be attached to the supply opening 42. A liquid supplied from the indeflator is passed through an inflation lumen 44 formed with the outer tube 30 and the first inner tube 50 to inflate the balloon 40.

Unlike the catheters 10, 10a, 10b, 10c, in the case of the balloon catheter 10d, the distal end of the balloon catheter 10d can be fixed by supplying a liquid from the supply opening 42 through the inflation lumen 44 to inflate the balloon 40 to the wall of a blood vessel, bile duct, pancreatic duct and the like. When a first guide wire inserted into the first inner tube 50 or a second guide wire inserted into the second inner tube 60 is operated while maintaining this state, the backup properties of the first guide wire or the second guide wire can be improved because the distal end of the balloon catheter 10d does not move.

As described above, the catheters 10, 10a, 10b, 10c and the balloon catheter 10d have configurations where the second core wires 80, 80a, 80b can move to the optimal position depending on the external forces 1, 2, 3, 4 exerted at a curved region of a blood vessel, bile duct, pancreatic duct and the like when the catheters 10, 10a, 10b, 10c and the balloon catheter 10d are inserted up to the curved region of a blood vessel, bile duct, pancreatic duct and the like. Therefore, a risk can be reduced that the first core wires 70, 70a and the second core wires 80, 80a, 80b interfere each other to prevent the catheters 10, 10a, 10b, 10c and the balloon catheter 10d from bending along a blood vessel, bile duct, pancreatic duct and the like, resulting in breakage of any one or both of the first core wires 70, 70a and the second core wires 80, 80a, 80b.

Note that the first core wires 70, 70a are fixed to the outer tubes 30, 30a, respectively as shown in Fig. 2B in the above description, but the configuration is not limited to this. The first core wires 70, 70a may be fixed with any one or both of the first inner tube 50 and the second inner tube 60 instead of the outer tubes 30, 30a

Moreover, the cross sectional shapes of the first core wire 70 and the second core wire 80 are both circular as shown in Figs. 2A to 2C and Fig. 3 in the above description, but the shapes are not limited to this. For example, the cross sectional shapes of the first core wire 70 and the second core wire 80 may be either elliptical or rectangular. However, in order to allow the second core wires 80, 80a, 80b to smoothly move to the optimal position depending on the external forces 1, 2, 3, 4 exerted at a curved region of a blood vessel, bile duct, pancreatic duct and the like, it is preferred to have a portion where the cross sectional area of the second core wires 80, 80a, 80b is smaller than that of the first core wires 70, 70a.

### DESCRIPTION OF SYMBOLS

- 1, 2, 3, 4: External force
- 10, 10a, 10b, 10c: Catheter
- 10d: Balloon catheter
- 20: Connector
- 30, 30a: Outer tube
- 34: Proximal end of outer tube
- 40: Balloon
- 42: Supply opening
- 44: Inflation lumen
- 50: First inner tube
- 52: First distal end opening
- 54: First insertion opening
- 60: Second inner tube
- 62: Second distal end opening
- 64: Second insertion opening
- 70, 70a: First core wire
- 72: Distal end of first core wire
- 74: Proximal end of first core wire
- 80, 80a, 80b: Second core wire
- 82, 82a, 82b: Distal end of second core wire
- 84, 86: Arrow
- 90, 100, 110: Restriction part

## Claims

1. A catheter (10, 10a, 10b, 10c, 10d) comprising:
an outer tube (30, 30a);
a first inner tube (50) inserted into the outer tube;
a second inner tube (60) inserted into the outer tube (30, 30a) parallel to the first inner tube (50), the second inner tube (60) having an insertion opening (64) provided at a proximal end and a distal end opening (62) provided at a distal end of the second inner tube (60);
a first core wire (70, 70a) inserted between the outer tube (30, 30a) and the first inner tube (50), the first core wire (70, 70a) being fixed to the outer tube (30, 30a) or the first inner tube (50); and
a second core wire (80, 80a, 80b) inserted parallel to the first core wire (70, 70a), the second core wire (80, 80a, 80b) being capable of moving between the outer tube (30, 30a) and the first inner tube (50), **characterized in that**
the second inner tube (60) is inserted from the middle through the distal end of the catheter (10, 10a, 10b, 10c, 10d),
the first core wire (70) is longer than the second core wire (80, 80a, 80b), and
a distal end (72, 72a) of the first core wire (70, 70a) is located in a distal end side relative to the insertion opening (64) of the second inner tube (60) while a distal end (82, 82a, 82b) of the second core wire (80, 80a, 80b) is located in a proximal end side relative to the insertion opening (64) of the second inner tube (60).

2. The catheter (10, 10a, 10b, 10c, 10d) according to claim 1, comprising a portion where a cross sectional area of the second core wire (80, 80a, 80b) is smaller than that of the first core wire (70, 70a).

3. The catheter (10a, 10b, 10c, 10d) according to claim 1 or 2, wherein a restriction part (90, 100, 110) restricting a movement of the second core wire (80, 80a, 80b) toward a distal end is provided at the first core wire (70a) or the second core wire (80a, 80b).

4. The catheter (10b, 10c, 10d) according to claim 3, wherein the restriction part (100, 110) is provided at the second core wire (80a, 80b), and the restriction part (100, 110) is positioned in a proximal side relative to a proximal end (74) of the first core wire (70) or a proximal end of the outer tube (30a).

5. A balloon catheter (10d) comprising:
a catheter (10, 10a, 10b, 10c) according to any one of claims 1 to 4;
a balloon (40) welded to the outer tube (30a) and the first inner tube (50); and
a supply opening (42) for supplying a liquid into the balloon (40).

## Patentansprüche

1. Katheter (10, 10a, 10b, 10c, 10d) mit:
einem Außenrohr (30, 30a);
einem im Außenrohr eingefügten ersten Innenrohr (50);
einem im Außenrohr (30, 30a) eingefügten, zum ersten Innenrohr (50) parallelen zweiten Innenrohr (60), das an seinem proximalen Ende eine Einführöffnung (64) und an seinem distalen Ende eine distale Öffnung (62) aufweist;
einem zwischen dem Außenrohr (30, 30a) und dem ersten Innenrohr (50) eingefügten ersten Kerndraht (70, 70a), der am Außenrohr (30, 30a) oder ersten Innenrohr (50) befestigt ist; und
einem zum ersten Kerndraht (70, 70a) parallelen zweiten Kerndraht (80, 80a, 80b), der zwischen dem Außenrohr (30, 30a) und dem ersten Innenrohr (50) beweglich ist, **dadurch gekennzeichnet, dass**
das zweite Innenrohr (60) von der Mitte bis zum distalen Ende des Katheters (10, 10a, 10b, 10c, 10d) eingefügt ist,
der erste Kerndraht (70) länger ist als der zweite Kerndraht (80, 80a, 80b), und
das distale Ende (72, 72a) des ersten Kerndrahts (70, 70a) relativ zur Einführöffnung (64) des zweiten Innenrohrs (60) auf einer distalen Seite liegt, während das distale Ende (82, 82a, 82b) des zweiten Kerndrahts (80, 80a, 80b) relativ zur Einführöffnung (64) des zweiten Innenrohrs (60) auf einer proximalen Seite liegt.

2. Katheter (10, 10a, 10b, 10c, 10d) nach Anspruch 1 mit einem Bereich, in dem die Querschnittsfläche des zweiten Kerndrahts (80, 80a, 80b) kleiner ist als die Querschnittsfläche des ersten Kerndrahts (70, 70a).

3. Katheter (10a, 10b, 10c, 10d) nach Anspruch 1 oder 2, wobei an dem ersten Kerndraht (70a) oder zweiten Kerndraht (80a, 80b) ein Einschränkungsteil (90, 100, 110) vorgesehen ist, das eine Bewegung des zweiten Kerndrahts (80, 80a, 80b) hin zum distalen Ende einschränkt.

4. Katheter (10b, 10c, 10d) nach Anspruch 3, wobei das Einschränkungsteil (100, 110) an dem zweiten Kerndraht (80a, 80b) vorgesehen ist und relativ zum proximalen Ende (74) des ersten Kerndrahts (70) oder zum proximalen Ende des Außenrohrs (30a) auf einer proximalen Seite liegt.

5. Ballonkatheter (10d) mit:
einem Katheter (10, 10a, 10b, 10c) nach einem der Ansprüche 1 bis 4;
einem am Außenrohr (30a) und ersten Innenrohr (50) angeschweißten Ballon (40); und
einer Einspeiseöffnung (42) zum Einspeisen einer Flüssigkeit in den Ballon (40).

## Revendications

1. Cathéter (10, 10a, 10b, 10c, 10d) comprenant :
un tube externe (30, 30a) ;
un premier tube interne (50) inséré dans le tube externe ;
un second tube interne (60) inséré dans le tube externe (30, 30a) parallèle au premier tube interne (50), le second tube interne (60) comportant une ouverture d'insertion (64) située à une extrémité proximale et une ouverture d'extrémité distale (62) située à une extrémité distale du second tube interne (60) ;
un premier fil central (70, 70a) inséré entre le tube externe (30, 30a) et le premier tube interne (50), le premier fil central (70, 70a) étant fixé au tube externe (30, 30a) ou au premier tube interne (50) ; et
un second fil central (80, 80a, 80b) inséré parallèlement au premier fil central (70, 70a), le second fil central (80, 80a, 80b) étant capable de se déplacer entre le tube externe (30, 30a) et le premier tube interne (50), **caractérisé en ce que**
le second tube interne (60) est inséré à partir du milieu par l'intermédiaire de l'extrémité distale du cathéter (10, 10a, 10b, 10c, 10d),
le premier fil central (70) est plus long que le second fil central (80, 80a, 80b), et
une extrémité distale (72, 72a) du premier fil central (70, 70a) est située d'un côté d'extrémité distale par rapport à l'ouverture d'insertion (64) du second tube interne (60), tandis qu'une extrémité distale (82, 82a, 82b) du second fil central (80, 80a, 80b) est située d'un côté d'extrémité proximale par rapport à l'ouverture d'insertion (64) du second tube interne (60).

2. Cathéter (10, 10a, 10b, 10c, 10d) selon la revendication 1, comprenant une partie où une surface en coupe transversale du second fil central (80, 80a, 80b) est plus petite que celle du premier fil central (70, 70a).

3. Cathéter (10a, 10b, 10c, 10d) selon la revendication 1 ou 2, dans lequel une partie de restriction (90, 100, 110) limitant un mouvement du second fil central (80, 80a, 80b) vers une extrémité distale est fournie sur le premier fil central (70a) ou le second fil central (80a, 80b).

4. Cathéter (10b, 10c, 10d) selon la revendication 3, dans lequel la partie de restriction (100, 110) est fournie sur le second fil central (80a, 80b), et la partie de restriction (100, 110) est positionnée d'un côté proximal par rapport à une extrémité proximale (74) du premier fil central (70) ou une extrémité proximale du tube externe (30a).

5. Cathéter à ballon (10d) comprenant :
un cathéter (10, 10a, 10b, 10c) selon l'une quelconque des revendications 1 à 4 ;
un ballon (40) soudé au tube externe (30a) et au premier tube interne (50) ; et
une ouverture d'alimentation (42) servant à introduire un liquide dans le ballon (40).
